# EUROPÄISCHE PATENTSCHRIFT

(11) **EP 3 255 128 B1**
(45) Veröffentlichungstag und Bekanntmachung des Hinweises auf die Patenterteilung: **01.12.2021**
(21) Anmeldenummer: 17175075.5
(22) Anmeldetag: 08.06.2017
(51) Int. Cl.: C10M 105/42, C10M 105/44, C10N 20/02, C10N 30/02

(54) **KOMPLEXESTER UND IHRE VERWENDUNG**
COMPLEX ESTERS AND THEIR USE
ESTER COMPLEXE ET SON UTILISATION

(30) Priorität: 08.06.2016 EP 16173537
(43) Veröffentlichungstag der Anmeldung: 13.12.2017
(73) Patentinhaber: Peter Greven GmbH & Co. KG, 53902 Bad Münstereifel (DE)
(72) Erfinder: Stolz, Hermann Josef, 53902 Bad Münstereifel (DE); Mennicken-Meuthen, Martina, 52078 Aachen (DE); Huber, Wilhelm, 50226 Frechen (DE); Flügel, Nadine, 53842 Troisdorf (DE)
(74) Vertreter: dompatent von Kreisler Selting Werner - Partnerschaft von Patent- und Rechtsanwälten mbB

(56) Entgegenhaltungen:
- DE-A1- 1 644 859
- DE-A1- 2 705 089
- DE-A1- 4 222 341
- DE-B- 1 264 659
- GB-A- 659 103
- GB-A- 1 083 215

## Beschreibung

Die vorliegende Erfindung betrifft Komplexester, ihre Herstellung und Verwendung als Basisöle für Schmierstoffe sowie diese Schmierstoffe.

Natürliche Fette und Öle werden bereits seit Jahrtausenden als Schmierstoffe verwendet. Spätestens mit dem Beginn der Industrialisierung und daraufhin beständig komplexer werdender Technologien haben sich die Anwendungen von Schmierstoffen und die damit einhergehenden Anforderungen an diese erheblich erweitert. Immer spezifischer werdende Probleme haben so nach immer spezifischeren Lösungen verlangt.

Generell besteht der Zweck eines Schmierstoffs darin, Reibung zwischen in Kontakt stehenden Oberflächen zu reduzieren, so dass letztendlich die bei Bewegung der Oberflächen erzeugte Wärme reduziert wird. Weiterhin verfolgte Ziele können Kraftübertragung, Transport von Partikeln oder Erwärmen oder Abkühlen von Oberflächen sein.

Um den gewünschten Zweck zu erfüllen, weisen Schmierstoffe in der Regel die folgenden Eigenschaften auf:
- Hohe Siedetemperatur und niedrige Gefriertemperaturen, d.h. breite Temperaturbereiche, in denen der Schmierstoff flüssig ist
- Hoher Viskositätsindex, d.h. eine geringe Änderung der Viskosität in Abhängigkeit von der Temperatur
- Thermische und hydraulische Stabilität
- Hohes Demulgiervermögen
- Korrosionsschutz
- Geringe Oxidationsanfälligkeit.

Hauptkomponente eines Schmierstoffs ist das Basisöl. Zu dem Basisöl werden oftmals Additive gegeben, um gewünschte Eigenschaften exakt einstellen zu können. Bei solchen Additiven handelt es sich etwa um Antioxiantien, Detergenzien, Extreme Pressure (EP)-Additive, Entschäumer etc.

Synthetische Basisöle können strukturell grundsätzlich in vier Klassen eingeteilt werden: Monocarbonsäureester, Dicarbonsäureester, Polyolester und

Komplexester. Monocarbonsäureester sind auf Grund von niedriger Viskosität und niedrigem Dampfdruck generell eher schlechte Schmierstoffe. Polyolester zeichnen sich, da sie nicht über β-H-Atome verfügen, gegenüber Dicarbonsäureestern durch eine erhöhte chemische Stabilität aus. Komplexester verfügen generell über vielversprechende Eigenschaften, ihre Herstellung ist allerdings im Industriemaßstab aufwendig, insbesondere bei Verwendung von Fettsäuren und Alkoholen als Edukten.

Obwohl bereits eine Vielzahl von synthetischen Basisölen bekannt ist, besteht dennoch weiterhin Bedarf an alternativen Zusammensetzungen, die bevorzugt jedenfalls einen der genannten Nachteile überwinden.

DE 1 264 659 betrifft Schmiermittel für Flugzeuggasturbinen.

GB 659,103 betrifft Komplexester.

GB 1,083,215 betrifft synthetische Schmiermittel.

DE-OS 16 44 859 beschreibt synthetische Schmiermittel.

DE 42 22 341 A1 beschreibt Grundöle mit hohem Viskositätsindex.

Aufgabe der vorliegenden Erfindung ist es, einen alternativen Schmierstoff bereitzustellen, der bevorzugt gegenüber dem Stand der Technik verbesserte Eigenschaften aufweist.

Gelöst wird diese Aufgabe durch Bereitstellung des erfindungsgemäßen Komplexesters mit der allgemeinen Formel **I** 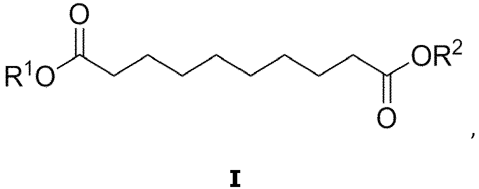 gemäß Anspruch 1.

Bereitgestellt wird also ein Komplexester. Dieser Komplexester ist ein Diester der Dicarbonsäure Sebacinsäure (Decandisäure, 1,8-Octandicarbonsäure, HO₂C(CH₂)₈CO₂H) mit zwei Alkoholen R¹OH und R²OH. Die beiden Alkohole R¹OH und R²OH, sogenannte Partialester, umfassen wiederum jeweils mindestens eine Esterbindung mit Fettsäuren.

In einer Ausführungsform zeichnen sich die erfindungsgemäßen Komplexester durch relativ kurzkettige Fettsäuren im Komplexester aus, wobei allerdings die verwendete Dicarbonsäure länger ist als die typischerweise verwendetete Adipinsäure (aber deutlich kürzer als Dimerfettsäuren). Überraschenderweise werden hierdurch einige Eigenschaften verbessert.

Im Sinne dieser Anmeldung kann eine Säure oder ein Alkohol auch den Säureteil oder den Alkoholteil eines Esters bezeichnen, d.h. es kann beispielsweise auch ein Teil eines Esters als Säureteil oder -komponente bezeichnet werden, obwohl keine freie Carboxygruppe (-CO₂H) oder Carboxylatgruppe (-CO₂⁻) vorhanden ist, sondern diese als funktionelle Gruppe Teil einer Esterbindung geworden sind. Dies gilt analog für Alkohole und freie Hydroxygruppen (-OH).

Ein Komplexester ist eine Verbindung, die eine mindestens zweiwertige Carbonsäure (Dicarbonsäure, Tricarbonsäure, etc.) umfasst, wobei die Carbonsäure mit mindestens zwei mindestens zweiwertigen Alkoholen verestert ist, wobei die Alkohole weitere Esterbindungen mit Fettsäuren aufweisen. Insbesondere ist jede Carboxygruppe der Carbonsäure mit mindestens zweiwertigen Alkoholen verestert.

Ein Partialester im Sinne dieser Anmeldung umfasst einen mindestens zweiwertigen Alkohol, aus dessen Veresterung mit einer mindestens zweiwertigen Carbonsäure ein Komplexester erhältlich ist, wobei der mindestens zweiwertige Alkohol mit mindestens einer Fettsäure, die von dem Partialester umfasst wird, verestert ist.

In einer weiteren Ausführungsform sind zwei oder mehr mindestens zweiwertige Carbonsäuren (Dicarbonsäure, Tricarbonsäure etc.) durch mindestens zweiwertige Alkohole verbrückt sind. Das heißt, die zwei oder mehr mindestens zweiwertigen Carbonsäuren sind kovalent durch verbrückende mindestens zweiwertige Alkohole verbunden, wie exemplarisch durch die Formel II dargestellt, wobei n > 0 ist, bevorzugt 0 < n < 10, mehr bevorzugt 0 < n < 5, noch mehr bevorzugt 0 < n < 3, am meisten bevorzugt n = 1 oder n = 2.

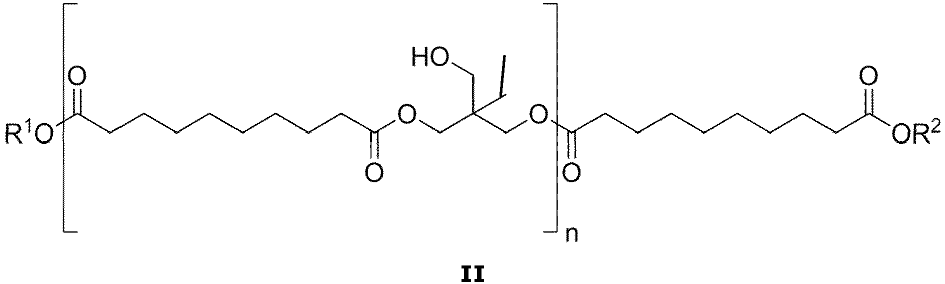

Bei **II** handelt es sich um Sebacinsäure. Der mindestens zweiwertige Alkohol ist bei **II** exemplarisch ein Trimethylolpropan (TMP)-Molekül, es ist aber prinzipiell jeder mindestens zweiwertige Alkohol geeignet. Es können im Fall n > 1 auch unterschiedliche, mindestens zweiwertige Alkohole in einem Molekül vorliegen.

Fettsäuren im Sinne dieser Anmeldung sind Mono- oder Polycarbonsäuren mit mindestens sechs C-Atomen, also Verbindungen, die mindestens eine Carboxylgruppe und verzweigte oder unverzweigte Kohlenstoffketten aufweisen. Eine Fettsäure im erfindungsgemäßen Sinne kann darüber hinaus noch Doppelbindungen, Hydroxy- oder Epoxygruppen aufweisen. Nur im Falle der ungesättigten Fettsäuren haben die Fettsäuren zwischen 6 und 28, bevorzugt 8 und 18 C-Atome. Soweit es sich um gesättigte Fettsäuren handelt, weisen diese höchstens eine Länge von 10 C-Atomen auf.

In einer Ausführungsform der Erfindung werden gesättigte Fettsäuren eingesetzt. Bevorzugt werden diese als Mischung eingesetzt. Diese Fettsäuren haben mindestens 6 und maximal 10 C-Atome. Geeignete Mischungen sind beispielsweise Fettsäuregemische enthaltend gesättigte C6, C8, C10 Fettsäuren oder Gemische enthaltend C8, C9, C10 Fettsäuren. Soweit ungesättigte Fettsäuren eingesetzt werden, können diese mehr C-Atome aufweisen.

Bevorzugt liegt der Schmelzpunkt der eingesetzten Fettsäuren unterhalb von 35 °C

Das molare Verhältnis von Fettsäuren zu Sebacinsäure ist kleiner 10:1, bevorzugt als 4:1 ist. Wenn alle eigesetzten Fettsäuren gesättigt sind, ist das bevorzugte molare Verhältnis von Fettsäuren zu Sebacinsäure kleiner 3:1. Das Molverhältnis ist mindestens 1:1 oder größer.

In einer weiteren bevorzugten Ausführungsform weist jeweils mindestens eine der Esterbindungen der Partialester R¹OH und R²OH in Bezug zur Alkoholfunktion kein β-H-Atom auf. In bevorzugten Ausführungsformen weisen alle Partialester in Bezug zur Alkoholfunktion kein β-H-Atom auf.

In einer weiteren bevorzugten Ausführungsform umfassen die Partialester R¹OH und R²OH jeweils zwei Esterbindungen mit Fettsäuren.

In einer besonders bevorzugten Ausführungsform sind die Fettsäuren der Partialester R¹OH und R²OH unabhängig voneinander ausgewählt aus der Gruppe bestehend aus gesättigten, ungesättigten, unverzweigten und verzweigten Fettsäuren und Mischungen daraus.

In einer weiteren besonders bevorzugten Ausführungsform sind die Fettsäuren ausgewählt aus der Gruppe bestehend aus Fettsäuren, erhältlich aus tierischen oder pflanzlichen Ausgangsstoffen, etwa Rapsöl, Sojaöl, Rizinusöl, Olivenöl, Kokosnussöl, Palmöl, Kiefernöl oder Talg, bevorzugt Fettsäuren mit 8, 10 oder 18 C-Atomen sowie Mischungen daraus, mehr bevorzugt Octansäure (Caprylsäure), Decansäure (Caprinsäure) und Oleinsäure sowie Mischungen daraus.

Die Partialester R¹OH und R²OH sind unabhängig voneinander durch Veresterung von Polyolen ausgewählt aus der Gruppe bestehend aus Trimethylolpropan (TMP), Di-Trimethylolpropan, Neopentylglycol (NPG) Pentaeryhthrol, Dipentaerythritol, Glycerin oder Mischungen hiervon erhältlich. Die Verwendung von TMP ist besonders bevorzugt.

Bevorzugt ist das molare Verhältnis von Polyol zu Sebacinsäure kleiner als 4:1 ist, bevorzugt kleiner als 3:1 oder - insbesondere wenn nur gesättigte Fettsäuren verwendet werden kleiner als 2:1. Das Molverhältnis ist bevorzugt mindestens 1:1 oder größer.

In einer Ausführungsform beträgt die Viskosität des Komplexesters höchstens 2000 mm²/s, gemessen bei 40 °C.

Bei allen Angaben zur Viskosität handelt es sich, wenn nicht explizit anders angegeben, um nach DIN 51562-1:1999-01 mit dem Ubbelohde-Viskosimeter gemessene kinematische Viskositäten. Diese wird üblicherweise in der Einheit [mm²/s] angegeben.

In einer Ausführungsform stammt die verwendete Sebacinsäure aus nachwachsenden Rohstoffquellen.

Ein weiterer Gegenstand der Erfindung ist ein Verfahren zur Herstellung von Komplexestern mit der allgemeinen Formel **I**, erhältlich durch Veresterung von Sebacinsäure mit zwei Partialestern R¹OH und R²OH, die jeweils mindestens eine Esterbindung mit Fettsäuren umfassen, umfassend die Schritte
a. Bereitstellen von zwei Partialestern R¹OH und R²OH, die jeweils mindestens eine Esterbindung mit Fettsäuren umfassen.
b. Veresterung von Sebacinsäure mit den Partialestern.

Erfindungsgemäß werden also zunächst die Partialester hergestellt, um anschließend die erhaltenen Partialester mit Sebacinsäure zu verestern, während im Stand der Technik Komplexester teilweise versucht wurde, diese durch gleichzeitige Veresterung der entsprechenden Edukte zu erhalten.

Ein weiterer Gegenstand der Erfindung ist die Verwendung eines Komplexesters mit der allgemeinen Formel **I**, erhältlich durch Veresterung von Sebacinsäure mit zwei Partialestern R¹OH und R²OH, die jeweils mindestens eine Esterbindung mit Fettsäuren umfassen, als Basisöl für Schmierstoffe.

Weiterhin ist ein Schmierstoff Gegenstand der Erfindung, enthaltend einen Komplexester mit der allgemeinen Formel **I**, erhältlich durch Veresterung von Sebacinsäure mit zwei Partialestern R¹OH und R²OH, die jeweils mindestens eine Esterbindung mit Fettsäuren umfassen, und mindestens einen Zusatzstoff ausgewählt aus der Gruppe bestehend aus Antioxidantien, Entschäumern, Extreme Pressure (EP)-Additiven, Verschleißhemmern (antiwear additive), Pour Point-Erniedrigern und Mischungen daraus.

In einer bevorzugten Ausführungsform sind die Antioxidantien ausgewählt aus der Gruppe bestehend aus phenolischen Antioxidantien, insbesondere Additin^{®} RC 7110, Additin^{®} RC 7115, Additin^{®} RC 7120; aminischen Antioxidantien, insbesondere Additin^{®} RC 7001, Additin^{®} RC 7010, Additin^{®} RC 7130, Additin^{®} RC 7135 LD, IRGANOX^{®} L 06, IRGANOX^{®} L 57; Phosphonaten, insbesondere IRGAFOSS^{®} 168; und Mischungen daraus, und/oder sind die Entschäumer ausgewählt aus der Gruppe bestehend aus Polyacrylaten, insbesondere FOAM BAN^{®} 3633E; Silikonverbindungen, insbesondere FOAM BAN^{®} 149; Siloxanen, insbesondere FOAM BAN^{®} 169; tensidischen Entschäumern, insbesondere ILCO-LUBE AF 2; Wachsdispersionen, insbesondere TEGO^{®} Antifoam 2205, TEGO^{®} Antifoam MR 2124; und/oder sind die Extreme Pressure (EP)-Additive ausgewählt aus der Gruppe bestehend aus Phosphorothioaten, insbesondere IRGALUBE^{®} 232; N-heterozyklischen Verbindungen, insbesondere Additin^{®} RC 8210, Additin^{®} RC 8213, Additin^{®} RC 8220; und Mischungen daraus; und/oder sind die Verschleißhemmer (antiwear additive) ausgewählt aus der Gruppe bestehend aus Carbamaten, insbesondere Additin^{®} RC 6340; Dialkyldithiophosphaten, insbesondere Additin^{®} RC 3038, Additin^{®} RC 3045, Additin^{®} RC 3080 und Additin^{®} RC 3580; Phosphorsäurederivaten, insbesondere Additin^{®} RC 3680 und Additin^{®} RC 3760; und Mischungen daraus; und/oder sind die Pour Point-Erniedriger ausgewählt aus der Gruppe bestehend aus IRGAFLO^{®} 610 P, IRGAFLO^{®} 649 P und Mischungen daraus.

Mit Blick auf Nachhaltigkeit und Verwendung nachwachsender Rohstoffe werden heutzutage vermehrt Öle auf Basis von pflanzlichen Ausgangsstoffen verwendet, etwa Rapsöl, Sojaöl, Rizinusöl, Olivenöl, Kokosnussöl, Palmöl oder Kiefernöl. Weitere Vorteile liegen in ihrer hohen Anwenderfreundlichkeit bzw. niedrigen Giftigkeit, biologischer Abbaubarkeit und der Vermeidung von Akkumulation in der Nahrungskette. Negative Eigenschaften im Hinblick auf die Verwendung in Schmierstoffen umfassen für solche Öle jedoch die geringe thermische Stabilität und hohe Oxidationsanfälligkeit, häufig auf Grund von ungesättigten Fettsäuren, sowie unerwünschte Charakteristika bei niedrigen Temperaturen, die wiederum aus der Fraktion gesättigter Fettsäuren resultieren.

Die erfindungsgemäßen Komplexester basieren auf der bevorzugten Verwendung von Edukten aus nachwachsenden Rohstoffquellen und weisen somit eine erhöhte Nachhaltigkeit gegenüber konventionellen Bestandteilen von Schmierstoffen auf.

Überraschenderweise können durch die erfindungsgemäßen Komplexester jedoch einige der oben genannten Nachteile überwunden werden.

Üblicherweise wurden bisher, im Gegensatz zur Sebacinsäure, Dicarbonsäuren mit längeren Kohlenwasserstoffketten, d.h. größerer Anzahl an C-Atomen, oder solche mit kürzeren Kohlenwasserstoffketten, d.h. geringerer Anzahl an C-Atomen verwendet. Ein Beispiel für längerkettige Säuren ist die Dimerfettsäure, die im Mittel 36 C-Atome, darunter zwei Carboxylgruppen, aufweist, also eine kovalente Verbindung von zwei C18-Carbonsäuren ist. Bei dem Bindeglied zwischen den C18-Carbonsäuren kann es sich um eine einfache kovalente Bindung zwischen jeweils einem C-Atom der beiden Ketten handeln, oder um aliphatische, aromatische oder gemischt aliphatisch-aromatische Zyklen, an denen mehr als jeweils 1 C-Atom der beiden Ketten beteiligt sind. In der Regel enthalten Dimerfettsäuren Verunreinigungen, z.B. Trimerfettsäure.

Ein Beispiel für kürzerkettige Säuren ist die Adipinsäure (HO₂C(CH₂)₄CO₂H).

"Komplexester auf Basis von R-säure" bezeichnen im Sinne dieser Anmeldung Komplexester, deren Polycarbonsäure R-säure ist, wobei "R-" für eine beliebige Benennung steht, z.B. "Dimerfett", "Adipin", "Sebacin" etc.

Überraschenderweise wurde gefunden, dass Komplexester der Sebacinsäure hervorragende Werte im Hinblick auf den Pourpoint und den Viskositätsindex aufweisen. Dies gilt insbesondere gegenüber Komplexestern der Adipinsäure und der Dimerfettsäure. Dieser Befund ist überraschend, da die Kettenlänge der Sebacinsäure (zehn C-Atome) zwischen der der Adipinsäure (sechs C-Atome) und der der Dimerfettsäure (18 C-Atome) liegt.

Für alle untersuchten und in der zu den Beispielen 2 und 3 korrespondierenden Tabelle aufgeführten Komplexester wurde gefunden, dass mindestens einer der Parameter einen signifikant verbesserten Wert aufwies.

So wurde im Vergleich zu den korrespondierenden Komplexestern der Adipinsäure und der Dimerfettsäure ein erniedrigter Pourpoint für die Komplexester der Sebacinsäure gemessen. Vereinfacht ist der Pourpoint die Temperatur, bei der die Probe nach Abkühlung nicht mehr fließfähig ist + 3C°. Ein niedriger Pourpoint erlaubt eine Anwendung bei tiefen Temperaturen. Es wurde also für die erfindungsgemäßen Komplexester ein breiterer Temperaturbereich, in dem der Komplexester in flüssiger Form, das heißt flüssiger Phase, vorliegt, gemessen, so dass ein breiterer Anwendungsbereich zugänglich ist.

Weiterhin wurde im Vergleich zu den korrespondierenden Komplexestern der Adipinsäure und der Dimerfettsäure ein erhöhter Viskositätsindex für die Komplexester der Sebacinsäure gemessen. Es wurde also für die erfindungsgemäßen Komplexester eine geringere Änderung der Viskosität mit der Temperatur gemessen, so dass Anwendern mit geringer Toleranz für Viskositätsänderungen geeignete Schmierstoffe zur Verfügung gestellt werden.

Bei allen Angaben zum Viskositätsindex handelt es sich, wenn nicht explizit anders angegeben, um nach ISO 2909:2002 aus den bei 40 °C und 100 °C bestimmten kinematischen Viskositäten berechnete Viskositätsindizes. Der Viskositätsindex wird demnach als dimensionslose Größe angegeben. Hohe Werte des Viskositätsindex zeigen, dass geringe Viskositätsschwankungen bei unterschiedlichen Temperaturen beobachtet werden.

Die beschriebenen Vorteile des gesenkten Pourpoints, bestimmt nach ISO 3016:1994, und des erhöhten Viskositätsindex , bestimmt nach ISO 2909:2002, sind dabei unabhängig von der Viskositätsklasse, bestimmt nach DIN ISO 3448:2010-02. Der angegebene Wert für die Viskositätsklasse, z.B. ISO-VG 320, benennt hierbei die bei 40 °C gemessene Viskosität, bei ISO-VG 320 also 320 mm²/s. Die Herstellung von Komplexestern unterschiedlicher Viskositätsklassen aus identischen Edukten beruht, wie dem Fachmann bekannt ist, auf der Verwendung unterschiedlicher Mengenverhältnisse von zentraler Carbonsäure und Partialestern bzw. von Polyol und Fettsäuren der Partialester.

Die Erfindung wird durch die nachstehenden Beispiele näher erläutert.

### Beispiel 1:

### a) Synthese eines Sebacinsäure-TMP-C8/C10-Fettsäure-Komplexesters (gesättigter Sebacinkomplexester mit ISO-VG 46) Vergleichsbeispiel

73,4 g eines C8/C10 Fettsäuregemischs (58:42 Gew.-%) und 26,15 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 10,75 g Sebacinsäure und 0,1 g SnO als Katalysator zudosiert. Anschließend wird das Produkt bei 140 - 220 °C und 100 - 250 mbar solange verestert, bis die Säurezahl <1 mg KOH/g beträgt. Nach Entfernen des Katalysators erhält man 100 g Ester mit einer Viskosität von 46,7 mm²/s.
Molverhältnis Alkohol:Sebacinsäre:Fettsäure ist 3,67:1:8,86.

### b) Synthese eines Sebacinsäure-TMP-Oleinsäure-Komplexesters (ungesättigter Sebacinkomplexester mit ISO-VG 320)

67,67 g Oleinsäure und 20,25 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 19,55 g Sebacinsäure und 0,1 g SnO als Katalysator zudosiert. Anschließend wird das Produkt bei 140 - 210 °C und 100 - 200 mbar solange verestert, bis die Säurezahl < 1 mg KOH/g beträgt. Nach Entfernen des Katalysators erhält man 100 g Ester mit einer Viskosität von 334 mm²/s.
Molverhältnis Alkohol:Sebacinsäre:Fettsäure ist 1,56:1:2,50.

### c) Synthese eines Sebacinsäure-TMP-C8/C10-Fettsäure-Komplexesters (gesättigter Sebacinkomplexester mit ISO-VG 320) Vergleichsbeispiel

54,88 g eines C8/C10 Fettsäuregemischs (58:42 Gew.-%) und 29,44 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 26,69 g Sebacinsäure und 0,1 g SnO als Katalysator zudosiert. Anschließend wird das Produkt bei 140 - 220 °C und 100 - 200 mbar solange verestert, bis die Säurezahl <1 mg KOH/g beträgt. Nach Entfernen des Katalysators erhält man 100 g Ester mit einer Viskosität von 322 mm²/s.Molverhältnis Alkohol:Sebacinsäre:Fettsäure ist 1,66:1:2,67.

### d) Synthese eines Sebacinsäure-TMP-Oleinsäure-Komplexesters (ungesättigter Sebacinkomplexester mit ISO-VG 220)

68,54 g Oleinsäure und 19,61 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 19,66 g Sebacinsäure und 0,1 g SnO als Katalysator zudosiert. Anschließend wird das Produkt bei 140 - 210 °C und 100 - 200 mbar solange verestert, bis die Säurezahl < 1 mg KOH/g beträgt. Nach Entfernen des Katalysators erhält man 100 g Ester mit einer Viskosität von 220 mm²/s.
Molverhältnis Alkohol:Sebacinsäre:Fettsäure ist 1,50:1:2,52.

### e) Synthese eines Sebacinsäure-TMP-C8-C9-C10-Komplexesters (gesättigter Sebacinkomplexester mit ISO-VG 320) Vergleichsbeispiel

26,85 g C8-C10, 27,44 g C9 und 29,44 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 26,69 g Sebacinsäure und 0,1 g SnO als Katalysator zudosiert. Anschließend wird das Produkt bei 140 - 210 °C und 100 - 200 mbar solange verestert, bis die Säurezahl < 1 mg KOH/g beträgt. Nach Entfernen des Katalysators erhält man 100 g Ester mit einer Viskosität von 322 mm²/s.
Molverhältnis Alkohol:Sebacinsäre:Fettsäure ist 1,66:1:2,67.

### f) Synthese eines Sebacinsäure-TMP-C8-C9-C10-Komplexesters (gesättigter Sebacinkomplexester mit ISO-VG 220) Vergleichsbeispiel

30,58 g C8-C10, 31,24 g C9 und 28,06 g Trimethylolpropan werden in einem Dreihalskolben mit einem Intensivkühler und Thermometer vorgelegt und bei 130 - 200 °C und 100 - 250 mbar verestert, bis die Säurezahl des Reaktionsgemisches <1 mg KOH/g beträgt. Das Gemisch wird abgekühlt und 26,68 g Sebacinsäure und 0,1 g SnO als Katalysator zudosiert. Anschließend wird das Produkt bei 140 - 210 °C und 100 - 200 mbar solange verestert, bis die Säurezahl < 1 mg KOH/g beträgt. Nach Entfernen des Katalysators erhält man 100 g Ester mit einer Viskosität von 209 mm²/s.
Molverhältnis Alkohol:Sebacinsäre:Fettsäure ist 1,59:1:2,98.

### Beispiel 2:

### Vergleichsbeispiele

Für die Beipiele 1 a)-d) wurden entsprechende Komplexester mit entweder Adipinsäure und Dimerfettsäuren (erhältlich durch Oligomerisierung von mehrfach ungesättigten Fettsäuren) anstelle von Sebacinsäure hergestellt, wobei die in den Beispielen beschriebenen Verfahrensbedingungen wurden und gleich Molverhältnisse gewählt wurden.

### Beispiel 3:

### Bestimmung des Viskositätsindex

Der Viskositätsindex verschiedener Komplexester wurde gemäß ISO 2909:2002 berechnet, nachdem die kinematische Viskosität nach DIN 51562-1:1999-01 mit dem Ubbelohde-Viskosimeter gemessen worden war.

Verglichen wurden erfindungsgemäße Komplexester der Sebacinsäure und solche der Adipin- und Dimerfettsäure. Bei den verwendeten Fettsäuren handelte es sich, unabhängig von der verwendeten mindestens zweiwertigen Carbonsäure, im Fall der gesättigten Komplexester um Gemische von Caprylsäure und Caprinsäure, im Fall der ungesättigten Komplexester um Oleinsäure.

Es wurden die in Tabelle 1 aufgeführten Werte erhalten.

### Beispiel 4:

### Bestimmung des Pourpoints

Der Pourpoint verschiedener Komplexester wurde gemäß der ISO 3016:1994 bestimmt. Verglichen wurden erfindungsgemäße Komplexester der Sebacinsäure und solche der Adipin- und Dimerfettsäure. Bei den verwendeten Fettsäuren handelte es sich, unabhängig von der verwendeten zentralen, mindestens zweiwertigen Carbonsäure, im Fall der gesättigten Komplexester um Gemische von Caprylsäure und Caprinsäure, im Fall der ungesättigten Komplexester um Oleinsäure.

Es wurden die in Tabelle 1 aufgeführten Werte erhalten.

**Tabelle 1 Beispiele 1 c und 1 a sind Vergleichsbeispiele**

| Viskositätsklasse [ISO-VG] | Carbonsäure | Fettsäuren | Viskositätsindex | Pourpoint [°C] |
|---|---|---|---|---|
| 320 (Beispiel 1 b)) | Sebacinsäure | ungesättigt | 189 | -39 |
| 320 (Vergleichsbeispiel) | Adipinsäure | ungesättigt | 184 | -39 |
| 320 (Vergleichsbeispiel) | Dimerfettsäure | ungesättigt | 184 | -33 |
| | | | | |
| 320 (Beispiel 1 c)) | Sebacinsäure | gesättigt | 173 | -39 |
| 320 (Vergleichsbeispiel) | Adipinsäure | gesättigt | 162 | -33 |
| 320 (Vergleichsbeispiel) | Dimerfettsäure | gesättigt | [158/164] | [-33/-36] |
| | | | | |
| 220 (Beispiel 1 d)) | Sebacinsäure | ungesättigt | 186 | -39 |
| 220 (Vergleichsbeispiel) | Adipinsäure | ungesättigt | 182 | -33 |
| 220 (Vergleichsbeispiel) | Dimerfettsäure | ungesättigt | 152 | -36 |
| | | | | |
| 46 (Beispiel 1 a)) | Sebacinsäure | gesättigt | 184 | -54 |
| 46 (Vergleichsbeispiel) | Adipinsäure | gesättigt | 153 | -51 |

## Patentansprüche

1. Komplexester mit der allgemeinen Formel 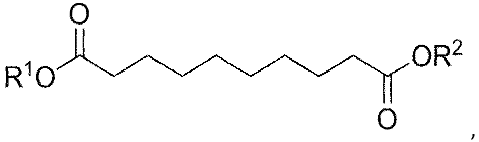
erhältlich durch Veresterung von Sebacinsäure mit zwei Partialestern R¹OH und R²OH, die jeweils mindestens eine Esterbindung mit Fettsäuren umfassen,
wobei die Fettsäuren, soweit sie gesättigt sind, höchstens 10 C-Atome aufweisen
wobei ungesättigte Fettsäuren eingesetzt werden, die 6 bis 28 C-Atome aufweisen,
wobei das molare Verhältnis von Fettsäuren zu Sebacinsäure kleiner als 10:1, aber mindestens 1:1 ist
wobei die Partialester R¹OH und R²OH unabhängig voneinander durch Veresterung von Polyolen ausgewählt aus der Gruppe bestehend aus Trimethylolpropan, Di-Trimethylolpropan, Neopentylglycol, Pentaerythritol, Dipentaerytrithol, Glycerin oder Mischungen hiervon erhältlich sind.

2. Komplexester nach Anspruch 1 mit der allgemeinen Formel 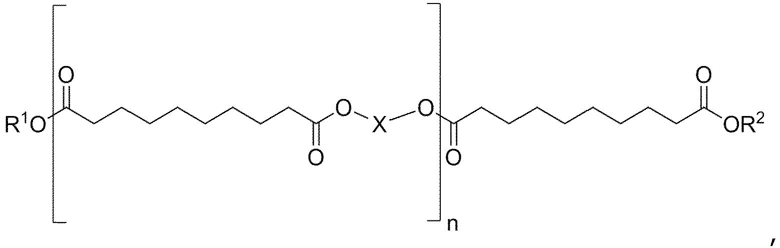 erhältlich durch Veresterung einer Verbindung aus n+1 Sebacinsäuremolekülen mit zwei Partialestern R¹OH und R²OH, die jeweils mindestens eine Esterbindung mit Fettsäuren umfassen, wobei n > 0 ist, wobei die n+1 Sebacinsäuremoleküle miteinander durch -O-X-O-Einheiten verbunden sind, und wobei es sich bei HO-X-OH um einen mindestens zweiwertigen Alkohol handelt und es sich bei den -O-X-O-Einheiten für den Fall n > 1 nicht um identische Einheiten handeln muss.

3. Komplexester nach Anspruch 2, wobei n 2 bis 9 beträgt.

4. Komplexester nach einem der Ansprüche 1 bis 3, wobei jeweils mindestens eine der Esterbindungen der Partialester R¹OH und R²OH in Bezug zur Alkoholfunktion kein β-H-Atom aufweist.

5. Komplexester nach einem der Ansprüche 1 bis 4, wobei die Fettsäuren der Partialester R¹OH und R²OH unabhängig voneinander ausgewählt sind aus der Gruppe bestehend aus gesättigten, ungesättigten, unverzweigten und verzweigten Fettsäuren und Mischungen daraus.

6. Komplexester nach einem der Ansprüche 1 bis 5, wobei die Fettsäuren ausgewählt sind aus der Gruppe bestehend aus Fettsäuren, erhältlich aus tierischen oder pflanzlichen Ausgangsstoffen, etwa Rapsöl, Sojaöl, Rizinusöl, Olivenöl, Kokosnussöl, Palmöl, Kiefernöl oder Talg, bevorzugt Fettsäuren mit 8, 10 oder 18 C-Atomen sowie Mischungen daraus, mehr bevorzugt Octansäure, Decansäure und Oleinsäure sowie Mischungen daraus.

7. Komplexester nach Anspruch 1, wobei das molare Verhältnis von Polyol zu Sebacinsäure kleiner als 4:1, aber mindestens 1:1 ist.

8. Verfahren zur Herstellung von Komplexestern nach einem der Ansprüche 1 bis 7 umfassend die Schritte
a. Bereitstellen von zwei Partialestern R¹OH und R²OH, die jeweils mindestens eine Esterbindung mit Fettsäuren umfassen.
b. Veresterung von Sebacinsäure mit den Partialestern.

9. Verwendung eines Komplexesters nach einem der Ansprüche 1 bis 7 als Basisöl für Schmierstoffe.

10. Schmierstoff, enthaltend einen Komplexester nach einem der Ansprüche 1 bis 7.

11. Schmierstoff nach Anspruch 10, weiterhin enthaltend mindestens einen Zusatzstoff ausgewählt aus der Gruppe bestehend aus Antioxidantien, Entschäumern, Extreme Pressure (EP)-Additiven, Verschleißhemmern (antiwear additive) und Pour Point-Erniedrigern.

## Claims

1. A complex ester of general formula 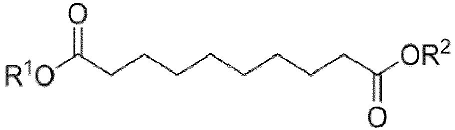
obtainable by the esterification of sebacic acid with two partial esters R¹OH and R²OH, each of which comprises at least one ester linkage with fatty acids,
wherein the fatty acids, when saturated, have at most 10 carbon atoms,
wherein unsaturated fatty acids having from 6 to 28 carbon atoms are employed,
wherein the molar ratio of fatty acids to sebacic acid is lower than 10:1, but at least 1:1,
wherein said partial esters R¹OH and R²OH are independently obtainable by the esterification of polyols selected from the group consisting of trimethylolpropane, ditrimethylolpropane, neopentyl glycol, pentaerythritol, dipentaerythritol, glycerol, or mixtures thereof.

2. The complex ester according to claim 1 of general formula 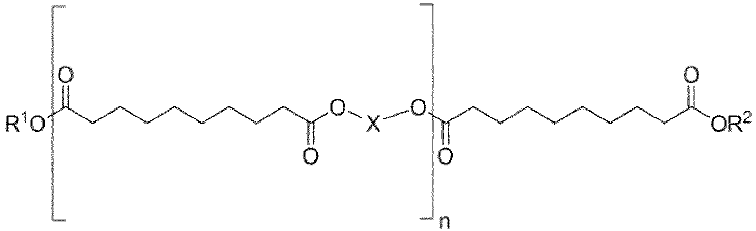 obtainable by the esterification of a compound consisting of n+1 sebacic acid molecules with two partial esters R¹OH and R²OH, each of which comprises at least one ester linkage with fatty acids, wherein n > 0, wherein said n+1 sebacic acid molecules are interlinked through -O-X-O- moieties, and wherein HO-X-OH is an at least dihydric alcohol, and said -O-X-O- moieties need not be identical moieties in a case where n > 1.

3. The complex ester according to claim 2, in which n is 2 to 9.

4. The complex ester according to any of claims 1 to 3, wherein at least one of the ester linkages of each of the partial esters R¹OH and R²OH does not have a β-hydrogen atom with respect to the alcohol function.

5. The complex ester according to any of claims 1 to 4, wherein the fatty acids of the partial esters R¹OH and R²OH are independently selected from the group consisting of saturated, unsaturated, unbranched and branched fatty acids and mixtures thereof.

6. The complex ester according to any of claims 1 to 5, wherein the fatty acids are selected from the group consisting of fatty acids obtainable from animal or vegetable starting materials, such as rapeseed oil, soybean oil, castor oil, olive oil, coconut oil, palm oil, pine oil or tallow, preferably fatty acids with 8, 10 or 18 carbon atoms, and mixtures thereof, more preferably octanoic acid, decanoic acid, and oleic acid, and mixtures thereof.

7. The complex ester according to claim 1, wherein the molar ratio of polyol to sebacic acid is lower than 4:1, but at least 1:1.

8. A process for preparing complex esters according to any of claims 1 to 7, comprising the steps of
a. providing two partial esters R¹OH and R²OH, each of which comprises at least one ester linkage with fatty acids,
b. esterifying sebacic acid with said partial esters.

9. Use of a complex ester according to any of claims 1 to 7 as a base oil for lubricants.

10. A lubricant comprising a complex ester according to any of claims 1 to 7.

11. The lubricant according to claim 10, further comprising at least one additive selected from the group consisting of antioxidants, defoamers, extreme pressure (EP) additives, antiwear additives, and pour point depressants.

## Revendications

1. Ester complexe de formule générale 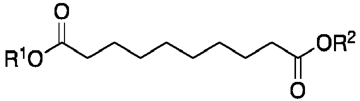
pouvant être obtenu par estérification de l'acide sébacique avec deux esters partiels R¹OH et R²OH, lesquels comprennent respectivement au moins une liaison ester avec des acides gras,
dans lequel les acides gras, pour autant qu'ils soient saturés, comportent au maximum 10 atomes de carbone,
dans lequel des acides gras insaturés comportant 6 à 28 atomes de carbone sont utilisés,
dans lequel la proportion molaire d'acides gras par rapport à l'acide sébacique est inférieure à 10:1 mais d'au moins 1:1,
dans lequel les esters partiels R¹OH et R²OH peuvent être obtenus indépendamment l'un de l'autre par estérification de polyols sélectionnés parmi le groupe constitué par le triméthylolpropane, le ditriméthylolpropane, le néopentylglycol, le pentaérythritol, le dipentaérytrithol, le glycérol et des mélanges de ceux-ci.

2. Ester complexe selon la revendication 1 de formule générale 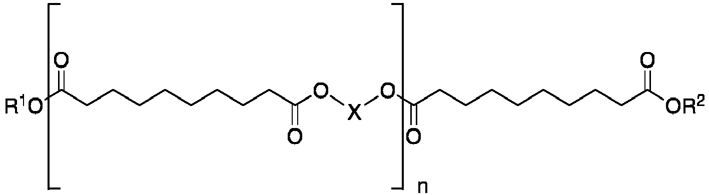 pouvant être obtenu par estérification d'un composé de n+1 molécules d'acide sébacique avec deux esters partiels R¹OH et R²OH, lesquels comprennent respectivement au moins une liaison ester avec des acides gras, dans lequel n > 0, dans lequel les n+1 molécules d'acide sébacique sont reliées l'une à l'autre par des unités -O-X-O- et dans lequel HO-X-OH est un alcool au moins divalent et dans le cas où n > 1 les unités -O-X-O- ne doivent pas être identiques.

3. Ester complexe selon la revendication 2, dans lequel n vaut entre 2 et 9.

4. Ester complexe selon l'une des revendications 1 à 3, dans lequel au moins une des liaisons esters des esters partiels R¹OH et R²OH ne comporte pas d'atome H en β par rapport à la fonction alcool.

5. Ester complexe selon l'une des revendications 1 à 4, dans lequel les acides gras des esters partiels R¹OH et R²OH sont sélectionnés indépendamment l'un de l'autre parmi le groupe constitué par les acides gras saturés, insaturés, non ramifiés et ramifiés et des mélanges de ceux-ci.

6. Ester complexe selon l'une des revendications 1 à 5, dans lequel les acides gras sont sélectionnés indépendamment l'un de l'autre parmi le groupe constitué par les acides gras pouvant être obtenus à partir de matériaux de départ animaux ou végétaux, par exemple huile de colza, huile de soja, huile de ricin, huile d'olive, huile de noix de coco, huile de palme, huile de pin ou suif, de préférence des acides gras à 8, 10 ou 18 atomes de carbone ainsi que des mélanges de ceux-ci, plus préférablement l'acide octanoïque, l'acide décanoïque et l'acide oléique ainsi que des mélanges de ceux-ci.

7. Ester complexe selon la revendication 1, dans lequel la proportion molaire de polyol par rapport à l'acide sébacique est inférieure à 4:1 mais d'au moins 1:1.

8. Procédé de fabrication d'esters complexes selon l'une des revendications 1 à 7, comprenant les étapes
a. Préparation de deux esters partiels R¹OH et R²OH, lesquels comprennent respectivement au moins une liaison ester avec des acides gras.
b. Estérification d'acide sébacique avec les esters partiels.

9. Utilisation d'un ester complexe selon l'une des revendications 1 à 7 comme huile de base pour lubrifiant.

10. Lubrifiant contenant un ester complexe selon l'une des revendications 1 à 7.

11. Lubrifiant selon la revendication 10, contenant en outre au moins un additif sélectionné parmi le groupe constitué par les antioxydants, les agents antimousses, les additifs EP (Extreme Pressure), les inhibiteurs d'usure (antiwear additive) et les abaisseurs de point d'écoulement.
